# EUROPEAN PATENT APPLICATION

(11) **EP 4 710 906 A1**
(43) Date of publication of application: **18.03.2026**
(21) Application number: 24802599.1
(22) Date of filing: 13.03.2024
(51) Int. Cl.: A61F 13/534, A61F 13/496, A61F 13/15

(54) **PAPER DIAPER CORE, PAPER DIAPER EQUIPPED WITH SAME, AND METHOD FOR PREPARING SAME**

(30) Priority: 08.05.2023 CN 202310530101
(71) Applicant: SHANDONG THRIVE MATERNITY AND INFANT PRODUCTS CO., LTD, Shandong 277500 (CN)
(72) Inventor: SUN, Feng, Zaozhuang, Shandong 277500 (CN); LIANG, Jinpan, Zaozhuang, Shandong 277500 (CN); WANG, Xishan, Zaozhuang, Shandong 277500 (CN)
(74) Representative: Cabinet Beau de Loménie
(86) International application number: PCT/CN2024/081398
(87) International publication number: WO 2024/230311

(57) **Abstract**

The present invention relates to a diaper core, a diaper equipped with the diaper core, and a method for preparing the same. The diaper core comprises: a wrapping fabric comprising a wrapping fabric lower surface, a wrapping fabric side surface, and a wrapping fabric upper surface that are integrally connected to form a receiving cavity inside the wrapping fabric; at least one layer of fluffy fabric disposed inside the receiving cavity and above the wrapping fabric lower surface, the middle of the fluffy fabric being provided with an opening of a predetermined length and predetermined width, and the fluffy fabric being attached with polymer particles; and a non-woven fabric disposed in the receiving cavity and located above the at least one layer of fluffy fabric; wherein the upper surface of the wrapping fabric and the non-woven fabric are recessed downwards at a position corresponding to the opening of the fluffy fabric until they are connected to the wrapping fabric lower surface, thereby forming a wrapping fabric concave part on the wrapping fabric upper surface and a non-woven fabric concave part on the non-woven fabric. The present invention improves capacity and rate of the diaper core to absorb urine, thereby enhancing hygiene and user experience of the diaper.

## Description

### Technical Field

The present invention relates to the field of diapers, and more particularly to a diaper core, a diaper equipped with such a diaper core, and a method for preparing the same.

### Background Art

Diapers are widely used by infants, young children, and patients who are unable to care for themselves. Existing diapers have a smooth surface, which easily causes urine and other substances to accumulate on the surface (i.e., the surface layer) of the diaper. The fluffy fabric inside the diaper, which is capable of absorbing large amounts of urine, cannot be fully utilized, resulting in poor and slow absorption of urine. This causes urine to accumulate on the surface layer, which is in direct contact with the user's skin, leading to a poor user experience.

The information disclosed in the background section of the present invention is only intended to enhance an understanding of the general background of the present invention, and should not be construed as an admission or suggestion in any form that such information constitutes prior art known to those skilled in the art.

### Summary of the Invention

The present invention aims to provide a diaper core, a diaper equipped with the diaper core, and a method for preparing the same. The diaper core can improve capacity and rate to absorb urine, thereby enhancing the hygiene and user experience of the diaper.

According to a first aspect of the present invention, there is provided a diaper core, comprising: a wrapping fabric comprising a wrapping fabric lower surface, a wrapping fabric side surface, and a wrapping fabric upper surface that are integrally connected to form a receiving cavity inside the wrapping fabric; at least one layer of fluffy fabric disposed in the receiving cavity and located above the wrapping fabric lower surface, the middle of the fluffy fabric being provided with an opening with a predetermined length and predetermined width, and the fluffy fabric being attached with polymer particles; and a non-woven fabric disposed inside the receiving cavity and located above the at least one layer of fluffy fabric; wherein the non-woven fabric is recessed downwards at a position corresponding to the opening of the fluffy fabric until it is connected to the wrapping fabric lower surface, thereby forming a non-woven fabric concave part on the non-woven fabric.

Preferably, the diaper core further comprises: absorbent cotton disposed between the non-woven fabric and the at least one layer of fluffy fabric, or disposed below the at least one layer of fluffy fabric, and having an opening corresponding to the opening of the fluffy fabric.

Preferably, the wrapping fabric upper surface is recessed downwards at a position corresponding to the opening of the fluffy fabric until it is connected to the wrapping fabric lower surface, thereby forming a wrapping fabric concave part on the wrapping fabric.

Preferably, the fluffy fabric is provided with two layers, both of which are provided with openings.

Preferably, the two layers of fluffy fabric comprise a first fluffy fabric and a second fluffy fabric which is closer to the non-woven fabric than the first fluffy fabric, a width of the opening of the second fluffy fabric is the same as that of the first fluffy fabric, and a length of the opening of the second fluffy fabric is shorter than that of the first fluffy fabric.

According to a second aspect of the present invention, there is provided a diaper, which is equipped with a surface layer and a diaper core according to the first aspect, wherein the surface layer is arranged above the diaper core.

Preferably, the surface layer is recessed downwards at a position corresponding to the opening to form a surface layer concave part.

According to a third aspect of the present invention, there is provided a method for preparing a diaper core, comprising steps of: a) providing a wrapping fabric and spraying glue on the wrapping fabric; b) preparing a first fluffy fabric with a first opening; c) placing the first fluffy fabric flat above the wrapping fabric; d) laying polymer particles on the first fluffy fabric and spraying glue on the first fluffy fabric; e) placing the non-woven fabric flat above the first fluffy fabric and spraying glue on the non-woven fabric; f) folding the wrapping fabric to form a receiving cavity that can wrap the first fluffy fabric and the non-woven fabric, and after folding, the wrapping fabric comprising a wrapping fabric lower surface, a wrapping fabric side surface, and a wrapping fabric upper surface that are integrally connected; g) pressing the wrapping fabric upper surface from top to bottom, so that the wrapping fabric upper surface and the non-woven fabric are recessed downwards at a position corresponding to the first opening until they are connected to the wrapping fabric lower surface, thereby forming a wrapping fabric concave part on the wrapping fabric upper surface, and forming a non-woven fabric concave part on the non-woven fabric, and connecting the wrapping fabric concave part and the non-woven fabric concave part to the wrapping fabric lower surface.

Preferably, preparing the first fluffy fabric with the first opening specifically comprises steps of: b1) providing a flat first fluffy fabric; b2) cutting the first fluffy fabric in the middle along an edge of a to-be-cut area with a predetermined length and predetermined width using a cutting blade, and removing the to-be-cut area to form the first opening.

Preferably, before step e), the method for preparing a diaper core further comprises steps of: h) preparing a second fluffy fabric with a second opening; i) placing the second fluffy fabric with the second opening flat above the first fluffy fabric; j) laying polymer particles on the second fluffy fabric and spraying glue on the second fluffy fabric; wherein step e) specifically comprises: placing the non-woven fabric flat above the second fluffy fabric that is above the first fluffy fabric.

Preferably, before step e), the method for preparing a diaper core further comprises steps of: k) preparing absorbent cotton with a third opening; l) placing the absorbent cotton with the third opening flat above or below the first fluffy fabric and spraying glue on the absorbent cotton; wherein step e) specifically comprises: placing the non-woven fabric flat above the first fluffy fabric and the absorbent cotton.

Preferably, before step e), the method for preparing a diaper core further comprises steps of: k) preparing absorbent cotton with a third opening; m) placing the absorbent cotton with the third opening flat above the second fluffy fabric, or between the first fluffy fabric and the second fluffy fabric, or below the first fluffy fabric, and spraying glue on the absorbent cotton; wherein step e) specifically comprises: placing the non-woven fabric flat above the first fluffy fabric, the second fluffy fabric, and the absorbent cotton.

Preferably, a width of the second opening is the same as that of the first opening, and a length of the second opening is shorter than that of the first opening.

Preferably, a width of the third opening is the same as that of the second opening, and a length of the third opening is the same as that of the second opening.

According to a fourth aspect of the present invention, there is provided a method for preparing a diaper, comprising: preparing a diaper core by the method for preparing a diaper core according to the third aspect; spraying glue on the wrapping fabric upper surface; laying a surface layer above the wrapping fabric upper surface; pressing the surface layer from top to bottom, so that the surface layer is recessed downwards at a position corresponding to the first opening to form a surface layer concave part, and connecting the surface layer concave part to the wrapping fabric lower surface.

When urine penetrates the diaper core, in addition to penetrating and diffusing from the wrapping fabric upper surface to the fluffy fabric, the concave part of the non-woven fabric can also guide the urine to the opening, and then the urine spreads around the opening, allowing the polymer particles attached to the fluffy fabric to absorb the urine more fully, improving the capacity and rate of the diaper core to absorb the urine, thereby enhancing the hygiene and user experience of the diaper.

The devices of the present invention have other characteristics and advantages that will be apparent from the accompanying drawings and subsequent embodiments incorporated herein, or will be described in detail in the accompanying drawings and subsequent embodiments, which together serve to explain the specific principles of the present invention.

### Brief Description of Drawings

Figure 1 is a sectional view of the diaper core in Example 1 of the present invention;
Figure 2A is a sectional view of the diaper core in Example 2 of the present invention;
Figure 2B is a sectional view of the diaper core in Example 2 of the present invention;
Figure 3 is a sectional view of the diaper core in Example 4 of the present invention;
Figure 4A is a sectional view of the diaper core in Example 5 of the present invention;
Figure 4B is a sectional view of the diaper core in Example 6 of the present invention;
Figure 4C is a sectional view of the diaper core in Example 7 of the present invention;
Figure 5A is a sectional view of the diaper core in Example 8 of the present invention;
Figure 5B is a sectional view of the diaper core in Example 9 of the present invention;
Figure 6 is a sectional view of a diaper provided by the present invention;
Figure 7 is a three-dimensional structural schematic view of a diaper provided by the present invention;
Figure 8 is a schematic view of an unfolded diaper of Figure 7 without the surface layer;
Figure 9 is a schematic flowchart of a first method for preparing a diaper core provided by the present invention;
Figure 10 is a schematic view of the positional relationship between polymer particles and a fluffy fabric;
Figure 11 is a schematic flowchart of a second method for preparing a diaper core provided by the present invention;
Figure 12 is a schematic flowchart of a third method for preparing a diaper core provided by the present invention;
Figure 13 is a schematic flowchart of a fourth method for preparing a diaper core provided by the present invention.

### List of reference numerals:

| | | | |
|---|---|---|---|
| 100: | wrapping fabric | 101: | wrapping fabric lower surface |
| 102: | wrapping fabric side surface | 103: | wrapping fabric upper surface |
| 104: | receiving cavity | 105: | wrapping fabric concave part |
| 201: | fluffy fabric | 202: | fluffy fabric |
| 203: | opening | 204: | opening |
| 300: | non-woven fabric | 301: | non-woven fabric concave part |
| 400: | absorbent cotton | 401: | opening |
| 500: | surface layer | 501: | surface layer concave part |
| 600: | polymer particles. | | |

It should be understood that the accompanying drawings are not necessarily drawn to scale, but rather present simplified representations of various features to illustrate the basic principles of the present invention. The specific design features disclosed in the present invention, including specific dimensions, directions, positions, and shapes, will be partially determined by the specific application and usage environment.

In these drawings, like reference numerals refer to the same or equivalent parts of the present invention throughout the drawing.

### Detailed Description of Embodiments

Reference will now be made in detail to various embodiments of the present invention, examples of which are presented in the accompanying drawings and described as follows. Although the present invention is described in conjunction with exemplary embodiments, it should be understood that this description is not intended to limit the present invention to these exemplary embodiments. On the contrary, the present invention aims to cover not only these exemplary embodiments, but also various substitutions, modifications, equivalents, and other embodiments that can be included within the spirit and scope of the present invention as defined by the appended claims.

It should be understood that when a component is referred to as being "connected to" another component, the component may be directly connected to the other component or intervening component(s) may be present. On the contrary, when a component is referred to as being "directly connected" to another component, there is usually no intermediate component.

When a component is referred to as being "above" or "on" another component, the component may be in contact with the other component or intermediate component(s) may be present.

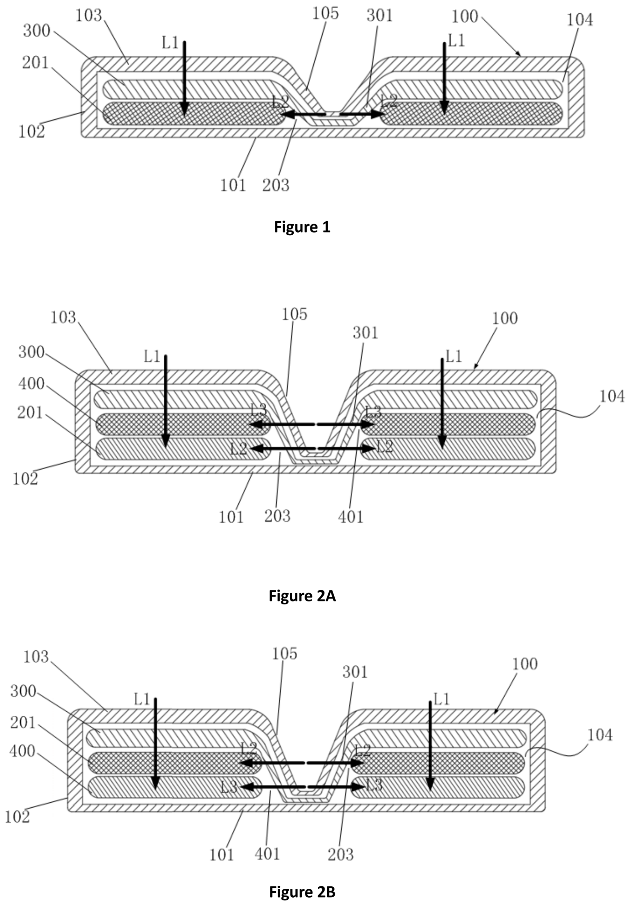

The upper side of the present invention is the upper side in Figures 1 to 6. When the diaper core is made into a diaper and worn on a person, the upper side is the side close to the skin.

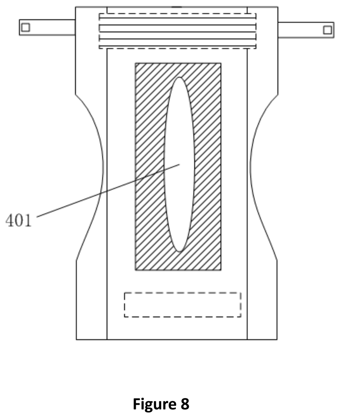

The diaper core according to embodiments of the present invention will be described below with reference to Figures 1 to 8.

As shown in Figure 1, a diaper core according to an embodiment of the present invention comprises: a wrapping fabric 100, at least one layer of fluffy fabric, and a non-woven fabric 300.

The wrapping fabric 100 comprises a wrapping fabric lower surface 101, a wrapping fabric side surface 102, and a wrapping fabric upper surface 103 that are integrally connected, so as to form a receiving cavity 104 inside the wrapping fabric 100.

At least one layer of fluffy fabric is disposed in the receiving cavity 104 and located above the wrapping fabric lower surface 101. The middle of the fluffy fabric is provided with an opening 203 with a predetermined length and predetermined width, and the fluffy fabric is attached with polymer particles (SAP particles). The polymer particles penetrate the fluffy fabric, that is, the polymer particles are present on the upper surface, in the interior and on the lower surface of the fluffy fabric. The polymer particles can be selected from polymer water-absorbent resin particles (SAP particles).

The non-woven fabric 300 is disposed inside the receiving cavity 104 and located above the at least one layer of fluffy fabric. The non-woven fabric 300 is used to conduct urine to facilitate absorption by the polymer particles and to diffuse urine towards the lower portion and both ends of the core.

The wrapping fabric upper surface 103 and the non-woven fabric 300 are recessed downwards at a position corresponding to the opening 203 of the fluffy fabric until they are connected to the wrapping fabric lower surface 101, thereby forming a wrapping fabric concave part 105 on the wrapping fabric upper surface 103, and forming a non-woven fabric concave part 301 on the non-woven fabric 300.

When urine penetrates the diaper core, in addition to penetrating and diffusing from the wrapping fabric upper surface 103 to the fluffy fabric (i.e., penetrating and diffusing in the direction of arrow L1), the wrapping fabric concave part 105 of the wrapping fabric upper surface 103 and the concave part 301 of the non-woven fabric 300 can also guide the urine to the opening 203, and then the urine diffuses around the opening 203 (i.e., diffusing in the direction of arrow L2), allowing the polymer particles attached to the fluffy fabric to absorb the urine more fully, improving the capacity and rate of the paper diaper core to absorb urine, thereby enhancing the hygiene and user experience of the diaper.

The non-woven fabric 300 can be selected from spunlace fabric, spunbond fabric, or spunmelt fabric.

It should be noted that all the accompanying drawings in the present invention only illustrate the approximate shapes of each layer and the approximate positional relationships between them. To better distinguish the boundaries between the layers, gaps are illustrated between the layers in Figures 1 to 6, while in actual products, there are no gaps between the layers.

### Example 1

As shown in Figure 1, the diaper core comprises a wrapping fabric 100, a fluffy fabric 201, and non-woven fabric 300.

The wrapping fabric 100 comprises a wrapping fabric lower surface 101, a wrapping fabric side surface 102, and a wrapping fabric upper surface 103 that are integrally connected, to form a receiving cavity 104 inside the wrapping fabric 100.

The fluffy fabric 201 is disposed in the receiving cavity 104 and located above the wrapping fabric lower surface 101. The middle of the fluffy fabric 201 is provided with an opening 203 with a predetermined length and predetermined width, and polymer particles are attached to the fluffy fabric 201, wherein the polymer particles penetrate the fluffy fabric 201.

The non-woven fabric 300 is disposed inside the receiving cavity 104 and located above the fluffy fabric 201.

The wrapping fabric upper surface 103 and the non-woven fabric 300 are recessed downwards at a position corresponding to the opening 203 until they are connected to the wrapping fabric lower surface 101, thereby forming a wrapping fabric concave part 105 on the wrapping fabric upper surface 103, and forming a non-woven fabric concave part 301 on the non-woven fabric 300.

When urine penetrates the diaper core, in addition to penetrating and diffusing from the wrapping fabric upper surface 103 to the fluffy fabric 201 (i.e., penetrating and diffusing in the direction of arrow L1), the wrapping fabric concave part 105 of the wrapping fabric upper surface 103 and the non-woven fabric concave part 301 of the non-woven fabric 300 can also guide the urine to the opening 203, and then the urine diffuses around the opening 203 (i.e., diffusing in the direction of arrow L2), allowing the polymer particles attached to the fluffy fabric 201 to absorb the urine more fully, thereby improving the capacity and rate of the diaper core to absorb the urine.

### Example 2

As shown in Figure 2A, the diaper core comprises a wrapping fabric 100, a fluffy fabric 201, a non-woven fabric 300, and absorbent cotton 400.

The wrapping fabric 100 comprises a wrapping fabric lower surface 101, a wrapping fabric side surface 102, and a wrapping fabric upper surface 103 that are integrally connected, to form a receiving cavity 104 inside the wrapping fabric 100.

The fluffy fabric 201 is disposed in the receiving cavity 104 and located above the wrapping fabric lower surface 101. The middle of the fluffy fabric 201 is provided with an opening 203 with a predetermined length and predetermined width, and polymer particles are attached to the fluffy fabric 201, wherein the polymer particles penetrate the fluffy fabric 201.

The absorbent cotton 400 is disposed between the non-woven fabric 300 and the fluffy fabric 201 and has an opening 401. The absorbent cotton 400 can also absorb urine. The absorbent cotton 400 may be a mixture of fluff pulp and polymer water-absorbing resin.

The non-woven fabric 300 is disposed inside the receiving cavity 104 and located above the absorbent cotton 400.

The wrapping fabric upper surface 103 and the non-woven fabric 300 are recessed downwards at positions corresponding to the openings 203 and 401 until they are connected to the wrapping fabric lower surface 101, thereby forming a wrapping fabric concave part 105 on the wrapping fabric upper surface 103, and forming a non-woven fabric concave part 301 on the non-woven fabric 300.

When urine penetrates the diaper core, in addition to penetrating and diffusing from the wrapping fabric upper surface 103 to the fluffy fabric 201 (i.e., penetrating and diffusing in the direction of arrow L1), the wrapping fabric concave part 105 of the wrapping fabric upper surface 103 and the non-woven fabric concave part 301 of the non-woven fabric 300 can also guide the urine to the openings 401 and 203, and then the urine diffuses around the opening 401 (i.e., diffusing in the direction of arrow L3) so that the absorbent cotton 400 can absorb the urine more fully, and diffuses around the opening 203 (i.e., diffusing in the direction of arrow L2) so that the polymer particles attached to the fluffy fabric 201 can absorb the urine more fully, thereby improving the capacity and rate of the diaper core to absorb the urine.

Compared to Example 1, Example 2 adds a layer of absorbent cotton 400, which can increase absorption capacity and improve absorption rate.

### Example 3

As shown in Figure 2B, the diaper core comprises a wrapping fabric 100, a fluffy fabric 201, a non-woven fabric 300, and absorbent cotton 400.

Example 3 differs from Example 2 in that the absorbent cotton 400 is located between the fluffy fabric 201 and the wrapping fabric lower surface 101.

### Example 4

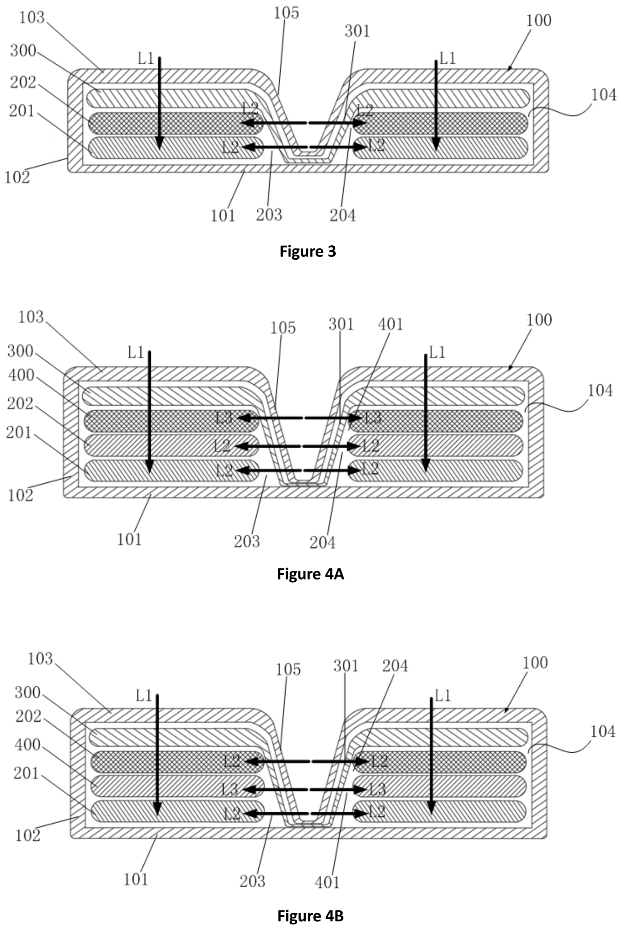

As shown in Figure 3, the diaper core comprises a wrapping fabric 100, a fluffy fabric 201, and a fluffy fabric 202, and a non-woven fabric 300.

The wrapping fabric 100 comprises a wrapping fabric lower surface 101, a wrapping fabric side surface 102, and a wrapping fabric upper surface 103 that are integrally connected, to form a receiving cavity 104 inside the wrapping fabric 100.

The fluffy fabric 201 is disposed in the receiving cavity 104 and located above the wrapping fabric lower surface 101. The middle of the fluffy fabric 201 is provided with an opening 203 with a predetermined length and predetermined width, and polymer particles are attached to the fluffy fabric 201, wherein the polymer particles penetrate the fluffy fabric 201.

The fluffy fabric 202 is disposed in the receiving cavity 104 and located above the fluffy fabric 201. The middle of the fluffy fabric 202 is provided with an opening 204 with a predetermined length and predetermined width, and polymer particles are attached to the fluffy fabric 201, wherein the polymer particles penetrate the fluffy fabric 202.

The non-woven fabric 300 is disposed inside the receiving cavity 104 and located above the fluffy fabric 202.

The wrapping fabric upper surface 103 and the non-woven fabric 300 are recessed downwards at positions corresponding to the openings 203 and 204 until they are connected to the wrapping fabric lower surface 101, thereby forming a wrapping fabric concave part 105 on the wrapping fabric upper surface 103, and forming a non-woven fabric concave part 301 on the non-woven fabric 300.

When urine penetrates the diaper core, in addition to penetrating and diffusing from the wrapping fabric upper surface 103 to the fluffy fabric 201 (i.e., penetrating and diffusing in the direction of arrow L1), the wrapping fabric concave part 105 of the wrapping fabric upper surface 103 and the non-woven fabric concave part 301 of the non-woven fabric 300 can also guide the urine to the openings 204 and 203, and then the urine diffuses around the openings 204 and 203 (i.e., diffusing in the direction of arrow L2), so that the polymer particles attached to the fluffy fabric 202 and 201 can absorb the urine more fully, thereby improving the capacity and rate of the diaper core to absorb the urine.

In the exemplary embodiment, the width of the opening 204 of the fluffy fabric 202 is the same as the width of the opening 203 of the fluffy fabric 201, and the length of the opening 204 of the fluffy fabric 202 is shorter than the length of the opening 203 of the fluffy fabric 201 to prevent urine at the opening 203 from escaping randomly to both sides too quickly, thereby preventing side leakage, and to prevent urine at the opening 203 from escaping randomly to the front and back, thereby preventing front and back leakage.

The relationship between the length of the opening 204 and the length of the opening 203 is not illustrated in the drawings.

In an exemplary embodiment, the size and thickness of the fluffy fabric 202 and the fluffy fabric 201 are the same.

In an exemplary embodiment, the width of the opening 203 and the opening 204 ranges from 5 to 70 mm, the length of the opening 203 ranges from 40 to 480 mm, and the length of the opening 204 ranges from 50 to 470 mm.

### Example 5

As shown in Figure 4A, the diaper core comprises a wrapping fabric 100, a fluffy fabric 201, and a fluffy fabric 202, absorbent cotton 400, and a non-woven fabric 300.

The wrapping fabric 100 comprises a wrapping fabric lower surface 101, a wrapping fabric side surface 102, and a wrapping fabric upper surface 103 that are integrally connected, to form a receiving cavity 104 inside the wrapping fabric 100.

The fluffy fabric 201 is disposed in the receiving cavity 104 and located above the wrapping fabric lower surface 101. The middle of the fluffy fabric 201 is provided with an opening 203 with a predetermined length and predetermined width, and polymer particles are attached to the fluffy fabric 201, wherein the polymer particles penetrate the fluffy fabric 201.

The fluffy fabric 202 is disposed in the receiving cavity 104 and located above the fluffy fabric 201. The middle of the fluffy fabric 202 is provided with an opening 204 with a predetermined length and predetermined width, and polymer particles are attached to the fluffy fabric 201, wherein the polymer particles penetrate the fluffy fabric 202.

The absorbent cotton 400 is disposed between the non-woven fabric 300 and the fluffy fabric 202, and has an opening 401.

The non-woven fabric 300 is disposed inside the receiving cavity 104 and located above the absorbent cotton 400.

The wrapping fabric upper surface 103 and the non-woven fabric 300 are recessed downwards at positions corresponding to the openings 203, 204, and 401 until they are connected to the wrapping fabric lower surface 101, thereby forming a wrapping fabric concave part 105 on the wrapping fabric upper surface 103, and forming a non-woven fabric concave part 301 on the non-woven fabric 300.

When urine penetrates the diaper core, in addition to penetrating and diffusing from the wrapping fabric upper surface 103 to the fluffy fabric 201 (i.e., penetrating and diffusing in the direction of arrow L1), the wrapping fabric concave part 105 of the wrapping fabric upper surface 103 and the non-woven fabric concave part 301 of the non-woven fabric 300 can also guide the urine to the openings 401, 204, and 203, and then the urine diffuses around the opening 401 (i.e., diffusing in the direction of arrow L3) so that the absorbent cotton 400 can absorb the urine more fully, and diffuses around the openings 204 and 203 (i.e., diffusing in the direction of arrow L2) so that the polymer particles attached to the fluffy fabric 202 and 201 can absorb the urine more fully, thereby improving the capacity and rate of the diaper core to absorb the urine.

### Example 6

As shown in Figure 4B, the diaper core comprises a wrapping fabric 100, a fluffy fabric 201, a fluffy fabric 202, absorbent cotton 400, and a non-woven fabric 300.

Example 6 differs from Example 5 in that the absorbent cotton 400 is located between the fluffy fabric 201 and the fluffy fabric 202.

### Example 7

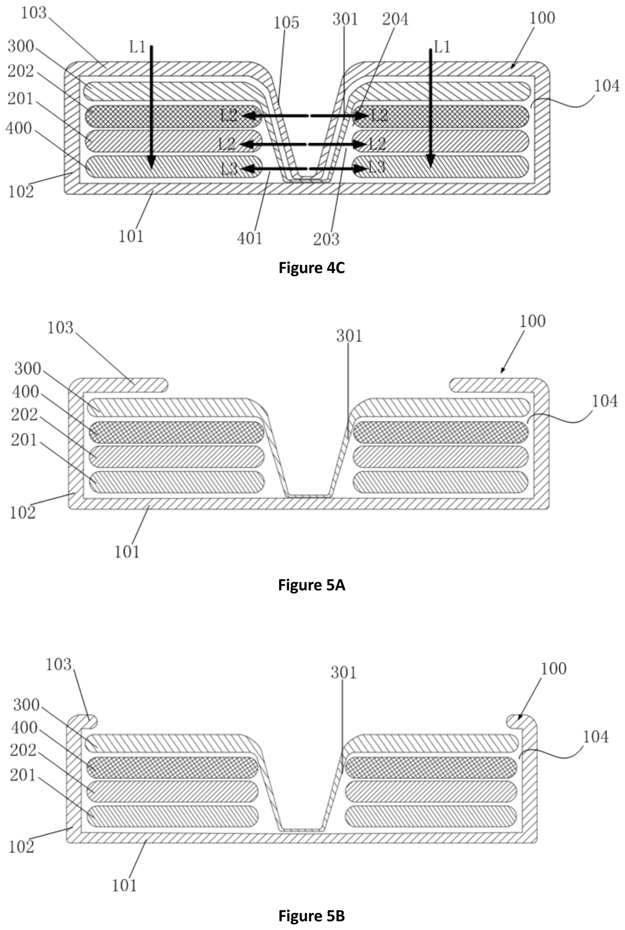

As shown in Figure 4C, the diaper core comprises a wrapping fabric 100, a fluffy fabric 201, a fluffy fabric 202, absorbent cotton 400, and a non-woven fabric 300.

Example 7 differs from Example 6 in that the absorbent cotton 400 is located between the fluffy fabric 201 and the wrapping fabric lower surface 101.

The number of layers of the fluffy fabric in the diaper core is adjustable.

### Example 8

As shown in Figure 5A, the diaper core comprises a wrapping fabric 100, a fluffy fabric 201, and a fluffy fabric 202, absorbent cotton 400, and a non-woven fabric 300.

Example 8 differs from Example 5 in that the wrapping fabric upper surface 103 does not completely cover the entire upper surface of the non-woven fabric 300, but only covers a portion (approximately half) of the upper surface of the non-woven fabric 300.

This covering relationship is also applicable to the embodiments of Figures 1, 2A, 2B, 3, 4B, and 4C.

### Example 9

As shown in Figure 5B, the diaper core comprises a wrapping fabric 100, a fluffy fabric 201, a fluffy fabric 202, absorbent cotton 400, and a non-woven fabric 300.

Example 9 differs from Example 5 in that the wrapping fabric upper surface 103 does not completely cover the entire upper surface of the non-woven fabric 300, but only covers the edge portion of the upper surface of the non-woven fabric 300.

This covering relationship is also applicable to the embodiments of Figures 1, 2A, 2B, 3, 4B, and 4C.

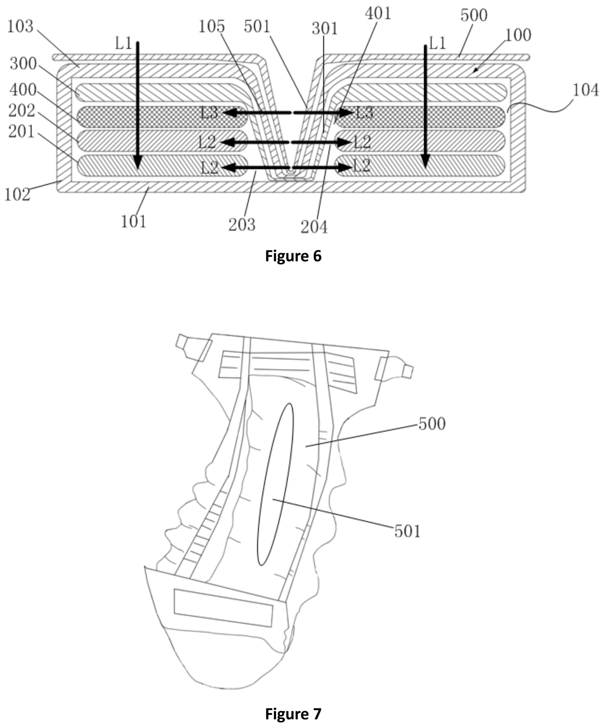

As shown in Figures 6 to 8, the embodiments of the present invention further provide a diaper, which comprises a surface layer 500 and the above-mentioned diaper core, wherein the surface layer is arranged above the diaper core. The diaper core in Figure 6 is exemplified by the diaper core in Example 5, and can be replaced with the diaper cores in Examples 1 to 4 and Examples 6 to 9. In addition, Figure 6 only illustrates the structure of the surface layer and the diaper core, and does not show the entire structure of the diaper.

The diaper herein includes not only traditional diapers but also pull-up pants.

In the exemplary embodiment, as shown in Figures 6 and 7, the surface layer 500 is recessed downwards at a position corresponding to the opening 203 to form a surface layer concave part 501. The surface layer 500 is a structure that directly contacts the user's skin.

To verify the effect of the diaper core in the embodiment of the present invention, control experiments are conducted on the diapers including Examples 1 to 7, wherein Experimental Groups 1 to 7 correspond to Examples 1 to 7, respectively. The only difference between the control group and the experimental group is the lack of corresponding concave parts and openings, with all other conditions being the same, wherein, "L" is the diffusion length displayed on the surface layer 500 during the absorption of 80ml of physiological saline. The longer the diffusion length displayed on the surface layer 500, the better the absorption effect of the diaper on the physiological saline is.

"t" is the time required to absorb 80ml of physiological saline. The shorter the time required, the faster the diaper core absorbs the physiological saline.

| | L (mm) | t (s) |
|---|---|---|
| Control Group 1 | 185 | 33 |
| Experimental Group 1 | 190 | 30 |
| Control group 2 | 187 | 34 |
| Experimental Group 2 | 198 | 30 |
| Control Group 3 | 188 | 34 |
| Experimental Group 3 | 203 | 29 |
| Control Group 4 | 190 | 32 |
| Experimental Group 4 | 208 | 29 |
| Control Group 5 | 190 | 30 |
| Experimental Group 5 | 207 | 27 |
| Control Group 6 | 195 | 29 |
| Experimental Group 6 | 213 | 25 |
| Control Group 7 | 200 | 29 |
| Experimental Group 7 | 219 | 24 |

As can be seen from the table, the diffusion lengths of the experimental groups with concave parts and openings exceed those of the corresponding control groups, and the absorption rates exceed those of the corresponding control groups.

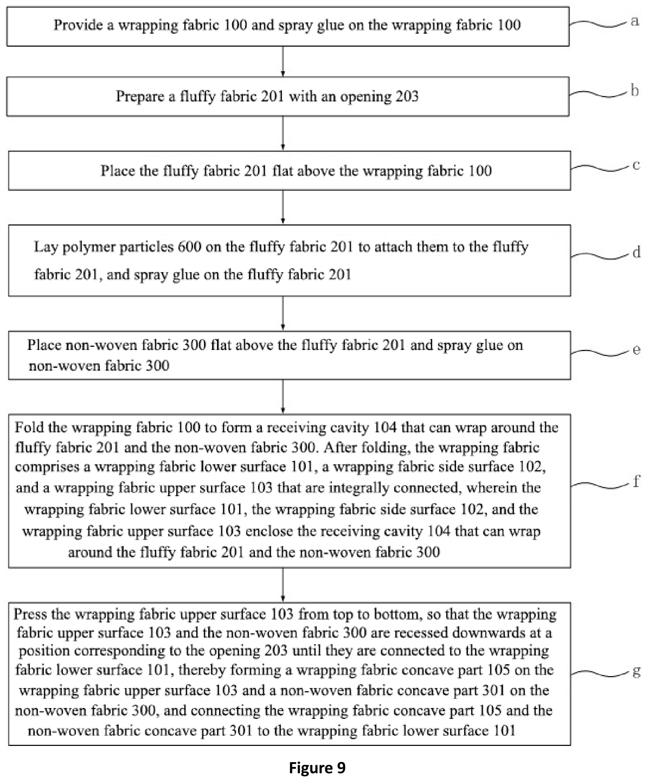

As shown in Figure 9, the present invention further provides a method for preparing a diaper core, comprising:
a) Providing a wrapping fabric 100 and spraying glue on the wrapping fabric 100.
b) Preparing a fluffy fabric 201 with an opening 203.
c) Placing the fluffy fabric 201 flat on the wrapping fabric 100.
d) Laying polymer particles 600 on the fluffy fabric 201 to attach the polymer particles 600 to the fluffy fabric 201, and spraying glue on the fluffy fabric 201.

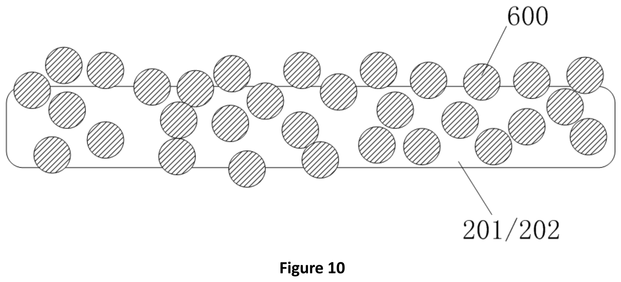

As shown in Figure 10, the polymer particles 600 penetrate downwards from the top of the fluffy fabric 201 to adhere to it, wherein some polymer particles 600 remain inside the fluffy fabric 201, some polymer particles 600 remain on the upper surface of the fluffy fabric 201, and some polymer particles 600 pass through the fluffy fabric 201 and adhere to the lower surface of the fluffy fabric 201.

e) Placing the non-woven fabric 300 flat on the fluffy fabric 201 and spraying glue on the non-woven fabric 300.

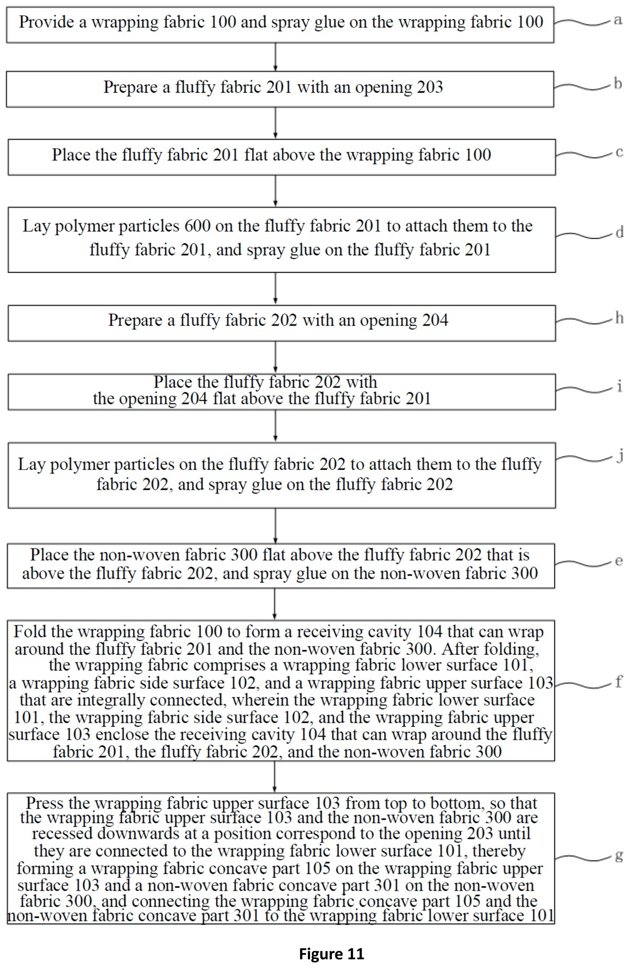

In all embodiments of Figures 9, 11 to 13, the glue can be selected from hot melt adhesive glue.

f) Folding the wrapping fabric 100 to form a receiving cavity 104 that can wrap the fluffy fabric 201 and the non-woven fabric 300, after folding, the wrapping fabric comprising a wrapping fabric lower surface 101, a wrapping fabric side surface 102, and a wrapping fabric upper surface 103 that are integrally connected, wherein the wrapping fabric lower surface 101, the wrapping fabric side surface 102, and the wrapping fabric upper surface 103 of wrapping fabric enclose the receiving cavity 104 that can wrap around the fluffy fabric 201 and the non-woven fabric 300.

g) Pressing the wrapping fabric upper surface 103 from top to bottom, so that the wrapping fabric upper surface 103 and the non-woven fabric 300 are recessed downwards at a position corresponding to the opening 203 until they are connected to the wrapping fabric lower surface 101, thereby forming a wrapping fabric concave part 105 on the wrapping fabric upper surface 103, and forming a non-woven fabric concave part 301 on the non-woven fabric 300, and connecting the wrapping fabric concave part 105 and the non-woven fabric concave part 301 to the wrapping fabric lower surface 101.

In this way, the diaper core as shown in Figure 1 is prepared.

In an exemplary embodiment, the steps of preparing the fluffy fabric 201 having the opening 203 specifically comprise:
b1) Providing a flat fluffy fabric 201.
b2) Cutting the fluffy fabric 201 in the middle along a to-be-cut area with a predetermined length and predetermined width using a cutting blade, and removing the to-be-cut area to form an opening 203.

In an exemplary embodiment, as shown in Figure 10, before step e), the method for preparing the diaper core further comprises:
h) Preparing a fluffy fabric 202 with an opening 204. The way for preparing the opening 204 is the same as that of the opening 203, and will not be repeated here.
i) Placing the fluffy fabric 202 with the opening 204 flat on the fluffy fabric 201.
j) Laying polymer particles on the fluffy fabric 202 to attach the polymer particles to the fluffy fabric 202, and spraying glue on the fluffy fabric 202.

Therein, step e) specifically comprises:
placing the non-woven fabric 300 flat on the fluffy fabric 202 that is on the fluffy fabric 201, and spraying glue on the non-woven fabric 300.

The receiving cavity 104 formed in the step f) can wrap around the fluffy fabric 201, the fluffy fabric 202, and the non-woven fabric 300.

The diaper core as shown in Figure 3 is obtained.

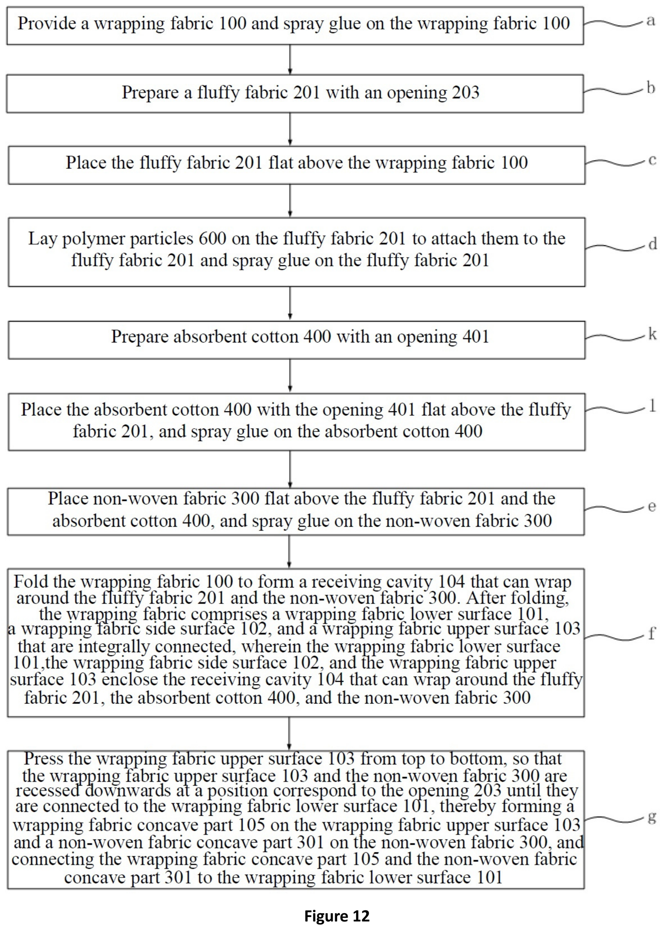

In an exemplary embodiment, as shown in Figure 12, before step e), the method for preparing the diaper core further comprises:
k) Preparing absorbent cotton 400 with an opening 401. The way for preparing the opening 401 is the same as that of the opening 203, and will not be repeated here.
I) Placing the absorbent cotton 400 with the opening 401 flat on the fluffy fabric 201, and spraying glue on the absorbent cotton 400.

Therein, step e) specifically comprises:
placing the non-woven fabric 300 flat on the fluffy fabric 201 and the absorbent cotton 400, and spraying glue on the non-woven fabric 300.

The receiving cavity 104 formed in step f) can wrap around the fluffy fabric 201, the absorbent cotton 400, and the non-woven fabric 300.

In this way, the diaper core as shown in Figure 2A is prepared.

In other embodiments, it is also possible to first place the absorbent cotton 400 with the opening 401 flat on the wrapping fabric 100, and then place the fluffy fabric 201 flat on the absorbent cotton 400 to prepare the diaper core as shown in Figure 2B.

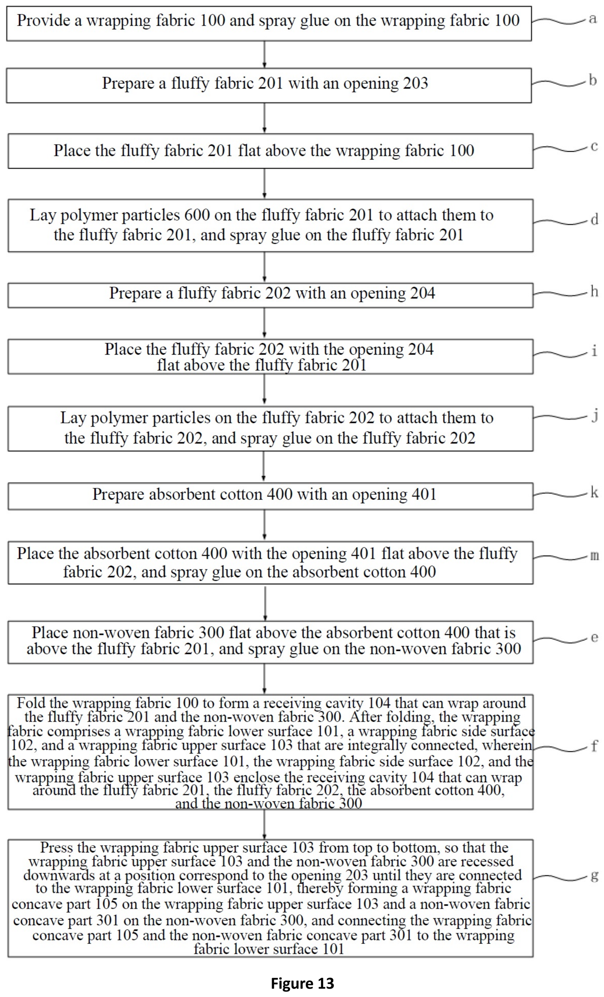

In an exemplary embodiment, as shown in Figure 13, after step j) and before step e), the method for preparing the diaper core further comprises:
k) Preparing absorbent cotton 400 with an opening 401.
m) Placing the absorbent cotton 400 with the opening 401 flat on the fluffy fabric 202, and spraying glue on the absorbent cotton 400.

Therein, step e) specifically comprises:
placing the non-woven fabric 300 flat on the absorbent cotton 400 that is on the fluffy fabric 201, and spraying glue on the non-woven fabric 300.

The receiving cavity 104 formed in step f) can wrap around the fluffy fabric 201, the fluffy fabric 202, the absorbent cotton 400, and the non-woven fabric 300.

In this way, the diaper core as shown in Figure 4A is prepared.

In another embodiment, it is also possible to place the absorbent cotton 400 with the opening 401 flat on the fluffy fabric 201, and then place the fluffy fabric 202 flat on the absorbent cotton 400, that is, the absorbent cotton 400 is disposed between the fluffy fabric 201 and the fluffy fabric 202.

In this way, the diaper core as shown in Figure 4B is prepared.

In yet another embodiment, it is also possible to place the absorbent cotton 400 with the opening 401 flat on the wrapping fabric 100, and then place the fluffy fabric 201 and the fluffy fabric 202 flat sequentially, that is, the absorbent cotton 400 is disposed below the fluffy fabric 201.

In this way, the diaper core as shown in Figure 4C is prepared.

In an exemplary embodiment, the width of the opening 204 is the same as the width of the opening 203, and the length of the opening 204 is shorter than the length of the opening 203.

In an exemplary embodiment, the width of opening 401 is the same as the width of opening 204, and the length of opening 401 is the same as the length of the opening 204.

The present invention further provides a method for preparing a diaper, comprising:
Preparing a diaper core by the method for preparing a diaper core according to any one of Figures 9, 11 and 13.

Spraying glue on the wrapping fabric upper surface.

Laying a surface layer 500 on the wrapping fabric upper surface 103.

Pressing the surface layer 500 from top to bottom, so that the surface layer 500 is recessed downwards at a position corresponding to the opening 203 to form a surface layer concave part 501, and connecting the surface layer concave part 501 to the wrapping fabric lower surface.

Taking the embodiments in Figures 4 and 5 as an example, the wrapping fabric upper surface can be first pressed from top to bottom to form a wrapping fabric concave part 105 on the wrapping fabric upper surface, and form a non-woven fabric concave part 301 on the non-woven fabric, then, the surface layer 500 can be laid, and then, the surface layer concave part 501 can be formed, that is, pressed twice.

In other embodiments, after folding the wrapping fabric 100, the surface layer 500 can be directly laid, and then the surface layer 500 can be pressed, forming the wrapping fabric concave part 105, the non-woven fabric concave part 301, and the surface layer concave part 501 at the same time, that is, pressed once.

The working process of the diaper according to the embodiments of the present invention will be described below with reference to the accompanying drawings.

The surface layer 500 directly contacts the user's skin.

The surface layer 500 absorbs urine, wherein in combination with Figure 6, the surface layer concave part 501 of the surface layer 500, the wrapping fabric concave part 105 of the wrapping fabric upper surface 103, and the non-woven fabric concave part 301 of the non-woven fabric 300 can also guide urine to the openings 203 and 204; then, the urine diffuses around the openings 203 and 204, allowing the polymer particles attached to the fluffy fabric 201 and 202 to absorb the urine more fully, thereby improving the hygiene and user experience of the diaper.

For convenience of interpretation and precise limitation of the accompanying claims, the terms "up", "down", "in", "out", "on", "below", "upside", "downside", "upward", "downward", "front", "back", "backward", "inside", "outside", "inward", "outward", "internal", "external", "internal", "external", "forwards", "backwards" are used to describe the features of the exemplary embodiments with reference to the positions of these features shown in the accompanying drawings.

The foregoing descriptions of specific exemplary embodiments of the present invention have been presented for purposes of illustration and exemplification. The preceding description is not intended to be exhaustive, nor is it intended to limit the invention to the precise form disclosed. Obviously, many modifications and variations are possible based on the above teachings. The purpose of selecting and describing exemplary embodiments is to explain the specific principles and practical applications of the present invention, so that others skilled in the art can implement and utilize various exemplary embodiments of the present invention, as well as various alternatives and modifications. The scope of the present invention is defined by the appended claims and their equivalents.

## Claims

1. A diaper core, **characterized in** comprising:
a wrapping fabric comprising a wrapping fabric lower surface, a wrapping fabric side surface, and a wrapping fabric upper surface that are integrally connected to form a receiving cavity inside the wrapping fabric;
at least one layer of fluffy fabric disposed in the receiving cavity and located above the wrapping fabric lower surface, the middle of the fluffy fabric being provided with an opening with a predetermined length and predetermined width, and the fluffy fabric being attached with polymer particles; and
a non-woven fabric disposed inside the receiving cavity and located above the at least one layer of fluffy fabric;
wherein the non-woven fabric is recessed downwards at a position corresponding to the opening of the fluffy fabric until it is connected to the wrapping fabric lower surface, thereby forming a non-woven fabric concave part on the non-woven fabric.

2. The diaper core according to claim 1, **characterized in that** the diaper core further comprises:
absorbent cotton disposed between the non-woven fabric and the at least one layer of fluffy fabric, or disposed below the at least one layer of fluffy fabric, and having an opening corresponding to the opening of the fluffy fabric.

3. The diaper core according to claim 1, **characterized in that** the wrapping fabric upper surface is recessed downwards at a position corresponding to the opening of the fluffy fabric until it is connected to the wrapping fabric lower surface, thereby forming a wrapping fabric concave part on the wrapping fabric.

4. The diaper core according to claim 1, **characterized in that** the fluffy fabric is provided with two layers, both of which are provided with openings.

5. The diaper core according to claim 4, **characterized in that** the two layers of fluffy fabric comprise a first fluffy fabric and a second fluffy fabric which is closer to the non-woven fabric than the first fluffy fabric, a width of the opening of the second fluffy fabric is the same as that of the first fluffy fabric, and a length of the opening of the second fluffy fabric is shorter than that of the first fluffy fabric.

6. A diaper, **characterized in that** it is equipped with a surface layer and a diaper core according to any one of claims 1-5, wherein the surface layer is arranged above the diaper core.

7. The diaper according to claim 6, **characterized in that** the surface layer is recessed downwards at a position corresponding to the opening to form a surface layer concave part.

8. A method for preparing a diaper core, **characterized in** comprising steps of:
a) providing a wrapping fabric and spraying glue on the wrapping fabric;
b) preparing a first fluffy fabric with a first opening;
c) placing the first fluffy fabric flat above the wrapping fabric;
d) laying polymer particles on the first fluffy fabric and spraying glue on the first fluffy fabric;
e) placing the non-woven fabric flat above the first fluffy fabric and spraying glue on the non-woven fabric;
f) folding the wrapping fabric to form a receiving cavity that can wrap the first fluffy fabric and the non-woven fabric, and after folding, the wrapping fabric comprising a wrapping fabric lower surface, a wrapping fabric side surface, and a wrapping fabric upper surface that are integrally connected;
g) pressing the wrapping fabric upper surface from top to bottom, so that the wrapping fabric upper surface and the non-woven fabric are recessed downwards at a position corresponding to the first opening until they are connected to the wrapping fabric lower surface, thereby forming a wrapping fabric concave part on the wrapping fabric upper surface, and forming a non-woven fabric concave part on the non-woven fabric, and connecting the wrapping fabric concave part and the non-woven fabric concave part to the wrapping fabric lower surface.

9. The method for preparing a diaper core according to claim 8, **characterized in that** the step of preparing the first fluffy fabric with the first opening specifically comprises steps of:
b1) providing a flat first fluffy fabric;
b2) cutting the first fluffy fabric in the middle along an edge of a to-be-cut area with a predetermined length and predetermined width using a cutting blade, and removing the to-be-cut area to form the first opening.

10. The method for preparing a diaper core according to claim 8, **characterized in that**, before step e), the method for preparing a diaper core further comprises steps of:
h) preparing a second fluffy fabric with a second opening;
i) placing the second fluffy fabric with the second opening flat above the first fluffy fabric;
j) laying polymer particles on the second fluffy fabric and spraying glue on the second fluffy fabric;
wherein step e) specifically comprises:
placing the non-woven fabric flat above the second fluffy fabric that is above the first fluffy fabric.

11. The method for preparing a diaper core according to claim 8, **characterized in that**, before step e), the method for preparing a diaper core further comprises steps of:
k) preparing absorbent cotton with a third opening;
l) placing the absorbent cotton with the third opening flat above or below the first fluffy fabric and spraying glue on the absorbent cotton;
wherein step e) specifically comprises:
placing the non-woven fabric flat above the first fluffy fabric and the absorbent cotton.

12. The method for preparing a diaper core according to claim 10, **characterized in that**, before step e), the method for preparing a diaper core further comprises steps of:
k) preparing absorbent cotton with a third opening;
m) placing the absorbent cotton with the third opening flat above the second fluffy fabric, or between the first fluffy fabric and the second fluffy fabric, or below the first fluffy fabric, and spraying glue on the absorbent cotton;
wherein step e) specifically comprises:
placing the non-woven fabric flat above the first fluffy fabric, the second fluffy fabric, and the absorbent cotton.

13. The method for preparing a diaper core according to claim 10, **characterized in that** a width of the second opening is the same as that of the first opening, and a length of the second opening is shorter than that of the first opening.

14. The method for preparing a diaper core according to claim 12, **characterized in that** a width of the third opening is the same as that of the second opening, and a length of the third opening is the same as that of the second opening.

15. A method for preparing a diaper, **characterized in** comprising:
preparing a diaper core by the method for preparing a diaper core according to any one of claims 8-14;
spraying glue on the wrapping fabric upper surface;
laying a surface layer above the wrapping fabric upper surface;
pressing the surface layer from top to bottom, so that the surface layer is recessed downwards at a position corresponding to the first opening to form a surface layer concave part, and connecting the surface layer concave part to the wrapping fabric lower surface.
